# EUROPEAN PATENT APPLICATION

(11) **EP 0 885 899 A1**
(43) Date of publication of application: **23.12.1998**
(21) Application number: 97310175.1
(22) Date of filing: 16.12.1997
(51) Int. Cl.: C07K 1/12

(54) **Method for obtaining peptide fragments by use of fluorinated organic acids**

(30) Priority: 16.12.1996 JP 336095/96
(71) Applicant: SEIKO INSTRUMENTS INC., Chiba-shi, Chiba 261 (JP)
(72) Inventor: Tsugita, Akira, Kashiwa-shi, Chiba (JP); Kawakami, Takao, Noda-shi, Chiba (JP); Kamo, Masaharu, Nagareyama-shi, Chiba (JP); Ataka, Tatsuaki, Seiko Instruments Inc., Chiba-shi, Chiba (JP); Sakuhara, Toshihiko, Seiko Instruments Inc., Chiba-shi, Chiba (JP); Uchida, Toyoaki, Seiko Instruments Inc., Chiba-shi, Chiba (JP)
(74) Representative: Sturt, Clifford Mark

(57) **Abstract**

This method obtains peptide fragments having ends of specific amino acids from a protein, which does not use an enzyme and thus is associated with minimum contamination. An organic acid represented by a general formula; CF3-(CF2) n-COOH, wherein n is an integer of 0 or larger, is allowed to act on a protein or peptide.

## Description

The present invention relates to a method for obtaining peptide fragments which is used for structural analysis of a protein or peptide.

Conventionally, a process for preparing peptide fragments having a specific amino acid or amino acid group at their ends by cleaving a protein has been used as a procedure for determining amino acid sequence in a protein, which is included in the technique for structural analysis of a protein. As the methods used in such process, the following methods have been reported.

### Enzymatic methods

1. A method for cleaving peptide bonds at the carboxy (C)-terminal side of lysines through digestion by lysylendopeptidase has been reported (Masaki, T., Tanabe, M., Nakamura, K., and Soejima, M. (1981) Biochem. Biophys. Acta, 66, 44-50).
2. A method for cleaving peptide bonds at the C-terminal side of glutamic acids through digestion by V8 protease has been reported (Drapeau, G.R., Boily, Y., and Houmard, J. (1972) J. Biol. Chem., 247, 6720).
3. A method for cleaving peptide bonds at the C-terminal side of arginines or lysines through digestion by trypsin has been reported (Koide Takehiko, Tanpakushitu no kagaku II (Seikagaku jikken koza 1), edited by Nippon Seikagaku-kai, Tokyo Kagaku Dojin, p255 (1976)).

### Chemical methods

4. A method for cleaving peptide bonds at the C-terminal side of methionines by cyanogen bromide (Gross, E., and Witkop, B., (1962) J. Biol. Chem., 237, 1856).
5. A method for cleaving peptide bonds at the amino (N)-terminal side of serines, threonines or glycines by concentrated hydrochloric acid has been reported (Sanger, F., and Thompson, E. O. P. (1953) Biochem. J. 53, 353-366).
6. A method for cleaving both the N-terminal and C-terminal sides of aspartic acids under an acidic conditions has been reported (Partridge, S.M., and Davis, H. F. (1950) Nature 165, 62-63) (Inglis, A. S. (1983) Methods Enzymol. 91, 324-332).
7. A method for cleaving peptide bonds at the C-terminal side of aspartic acids under an acidic conditions has been suggested (Inglis, A. S. (1983) Methods Enzymol. 91, 324-332).
8. A method for cleaving aspartylproline bonds under an acidic conditions has been suggested (Landon, M. (1977) Methods Enzymol. 47, 145-149).
9. It has been reported that as a side reaction of a consecutive degradation from the C-terminus of a protein or peptide using an organic acid, cleavage of N-terminal peptide bonds of serines and the C-terminal peptide bonds of aspartic acids are partially proceeded (Tsugita, A., Takamoto, K., Kamo, M., and Iwadate, H. (1992) Eur. J. Biochem. 206, 691-696, or Japanese Laid-open Patent Application No. HEI 5-52852).
   Precise analysis is sometimes difficult when conventional enzymatic methods are used, because the substrate specificity and enzymatic activity vary in dependence upon the target amino acid sequence, and unwanted cleavage of peptide bonds may occur due to contamination by other proteases. Furthermore these methods are inadequate for micro analysis applications because of contamination by undesirable amino acids or peptides as a result of autolysis or insufficient purification of the enzymes.

The above-described methods 1 to 8, including those using enzymes, are so-called liquid phase methods in which the sample and the solution containing the reagent are brought into direct contact, with the result that contaimination brought about by the solution is inevitable.

Furthermore such liquid phase methods require a troublesome process for removing the reagent solution after ther reaction. Additionally, in the above-mentioned method 9, the main reaction is a consecutive degradation from the C-terminus of the protein or peptide. Therefore this method is not suitable for cleaving a protein in such a manner as to obtain peptide fragments having specific amino acids or amino acid groups at their ends.

An object of the invention is to overcome or alleviate at least some of the above disadvantages.

The invention provides a method of obtaining pepetide fragments wherein a fluorinated organic acid preferably of general formula CF₃(CF₂)ₙ.COOH is reacted with a protein or a peptide, n being zero or an integer.

Preferably the organic acid is a perfluorinated acid, preferably a perfluorinated carboxylic acid.

If a perfuorinated carboxylic acid of general formula CF₃(CF₂)ₙ.COOH is used, preferably n is zero to 12, more preferably zero to 6, desirably zero, 1 or 2.

At least in preferred embodiments, the invention enables peptide fragments having specific terminal amino acids to be obtained.

Preferred features are defined in the dependent claims.

Embodiments of the invention are described below by way of example only with reference to Figures 1 to 31 of the accompanying drawings, wherein:
Figure 1 is a flowchart illustrating a method in accordance with the present invention;
Figure 2 is an illustration of apparatus useful for performing the above method;
Figure 3 is a plot of rate of degradation: HFBA (heptafluorobutyric acid) concentration for the reaction of HFBA with a hexadecapeptide of Sequence No. 1 at a reaction temperature of 50 degrees C ;
Figure 4 is a similar plot showing the corresponding results at a reaction temperature of 90 degrees C;
Fig.5 is a mass spectrogram showing the results of mass spectrometry of products produced by allowing HFBA to act on the hexadecapeptide of Sequence No.1;
Fig.6 is a mass spectrogram showing the results of mass spectrometry of products produced by allowing HFBA to act on the hexadecapeptide of Sequence No.1;
Fig. 7 is a mass spectrogram showing the results of mass spectrometry of products produced by allowing HFBA to act on the hexadecapeptide of Sequence No.1;
Fig. 8 is a mass spectrogram showing the results of mass spectrometry of products produced by allowing HFBA to act on the hexadecapeptide of Sequence No.1;
Fig. 9 is a mass spectrogram showing the results of mass spectrometry of products produced by allowing HFBA to act on the hexadecapeptide of Sequence No.2;
Fig. 10 is a mass spectrogram showing the results of mass spectrometry of products produced by allowing HFBA to act on the pentadecapeptide of Sequence No.3;
Fig. 11 is a mass spectrogram showing the results of mass spectrometry of products produced by allowing HFBA to act on the undecapeptide of Sequence No.4;
Fig. 12 is an illustration of electropherogram of products produced by allowing HFBA to act on ADH;
Fig. 13 is an illustration of electropherogram of products produced by allowing HFBA to act on CGMMV;
Fig. 14 is an illustration of electropherogram of products produced by allowing HFBA to act on TMV;
Fig. 15 is an illustration of electropherogram of products produced by allowing HFBA to act on TM-1;
Fig. 16 is a graph summarizing the results of amino acid analysis of products obtained by allowing HFBA to act on glycylthreonine and glycylserine;
Fig. 17 is an illustration of electropherogram of products obtained by allowing HFBA to act on ADH;
Fig. 18 is an illustration of electropherogram of products obtained by allowing HFBA to act on CGMMV;
Fig. 19 is an illustration of electropherogram of products obtained by allowing HFBA to act on TMV;
Fig. 20 is a mass spectrogram showing the results of mass spectrometry of products obtained by allowing HFBA to act on the hexadecapeptide of Sequence No.1;
Fig. 21 is a mass spectrogram showing the results of mass spectrometry of products obtained by allowing HFBA to act on the tridecapeptide of Sequence No.5;
Fig. 22 is a mass spectrogram showing the results of mass spectrometry of products obtained by allowing HFBA to act on the tridecapeptide of Sequence No.5;
Fig. 23 is a mass spectrogram showing the results of mass spectrometry of products obtained by allowing HFBA to act on the tridecapeptide of Sequence No.5;
Fig. 24 is an illustration of electropherogram of products obtained by allowing HFBA to act on cytochrome c;
Fig. 25 is an illustration of electropherogram of products obtained by allowing HFBA to act on myoglobin;
Fig. 26 is an illustration 'of electropherogram of products obtained by allowing HFBA to act on TM-1;
Fig. 27 is an illustration of electropherogram of products obtained by allowing HFBA to act on H2A;
Fig. 28 is a mass spectrogram showing the results of mass spectrometry of products obtained by allowing HFBA to act on the dodecapeptide of Sequence No.6;
Fig. 29 is a mass spectrogram showing the results of mass spectrometry of products obtained by allowing HFBA to act on the tridecapeptide of Sequence No.5;
Fig. 30 is an illustration of electropherogram of products obtained by allowing HFBA to act on CA-II; and
Fig. 31 is an illustration of electropherogram of products obtained by allowing HFBA to act on CGMMV.

### DETAILED DESCRIPTION OF THE INVENTION

The present invention will be further illustrated by the following examples.

### Example 1

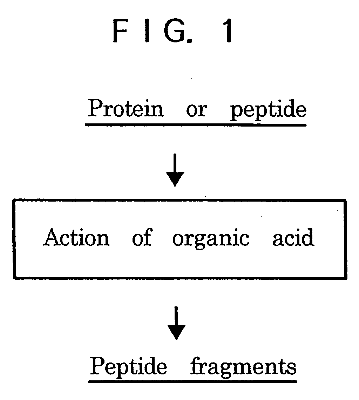

Fig. 1 is a flowchart representing the present invention. Peptide fragments are obtained by allowing an organic acid represented by the following general formula; CF3- (CF2) n-COOH, wherein n is an integer 0 or larger, on a protein or peptide.

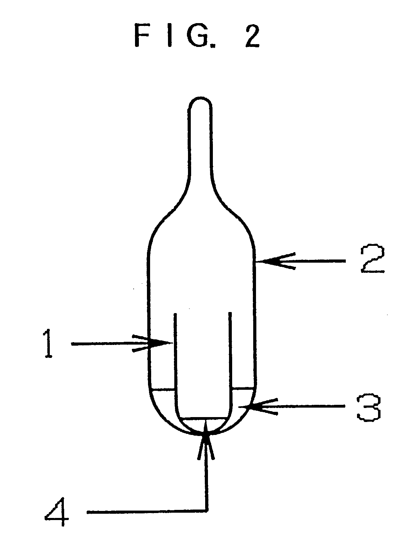

Fig. 2 shows the experimental methods of the present invention. First, sample solution containing a protein or peptide sample was placed in small-sized test tube 1 then allowed to dry. Separately, aqueous solution 3 containing an organic acid represented by general formula; CF3-(CF2) n-COOH, wherein n is an integer 0 or larger, which was a reaction reagent acting on the sample was placed in test tube 2. In the test tube 2, the above-described small-sized test tube 1 containing the dry sample 4 was placed. The test tube 2 was sealed in vacuo using a vacuum pump. The test tube 2 was then heated to a suitable (constant) reaction temperature (eg 50 degrees C) to allow the acid and water to evaporate and, whilst in the vapour phase, to contact and react with the sample in test tube 1. After the reaction had completed, the upper part of the test tube 2 was cut off and the small test tube 1 was removed. The reagent and solvent were then removed by vacuum desiccation.

In the formula CF3-(CF2)n-COOH a preferred upper limit for n is 5. The resulting perfluorinated acids will be readily apparent. For example:-
Trifluoroacetic acid (n = 0)
Nonafluorovaleric acid (n = 3)
Undecafluorohexanoic acid (n = 4)

### Example 2

Protein or peptide degradation by allowing heptafluorobutyric acid (HFBA), one of the organic acids represented by the above-described general formula; CF3-(CF2) n-COOH, wherein n is a integral number 0 or larger, to act on will be explained. First an experiment with the following reaction conditions was performed.

### (Reaction conditions)

HFBA concentration; 0, 5, 10, 30, 50, 70 and 90%
   (aqueous solution including 5% dithiothreitol (DTT))
Reaction temperature; 50°C
Reaction time; 24 hours

As a sample, hexadecapeptide of Sequence No.1 (Glu-Ala-Asp-Lys-Ala-Asp-Val-Asn-Val-Leu-Thr-Lys-Ala-Lys-Ser-Gln) was used. Sample prepared according to the procedure described in Example 1 was analyzed using a mass spectrometer.

### (Conditions for mass spectrometry)

### Mass spectrometer: Double focusing mass spectrometer (Nihon Denshi)

| Measurement conditions: | |
|---|---|
| Accelerating voltage | 10 kV |
| Resolution | 1,000 |
| Ion source | FAB(fast atom bombardment) |
| Ionization gas | Xe |
| Ion mode | cation |
| FAB gun accelerating voltage | 6 kV |
| Detection unit | MULTIPLIER |
| Load voltage | -20 kV |
| Data processing system | DA5000 |
| Matrix glycerol:thioglycerol:m-nitrobenzyl alcohol = 1:1:1 | |

Procedure to prepare a sample:
1) Dry a sample in reduced pressure.
2) Dissolve the sample in 2 µl of 67 % acetic acid aqueous solution.
3) Apply 1 µl of matrix on the target.
4) Add 1 µl of the sample solution on the target, then mix them.
5) Introduce the sample solution into the ion source.

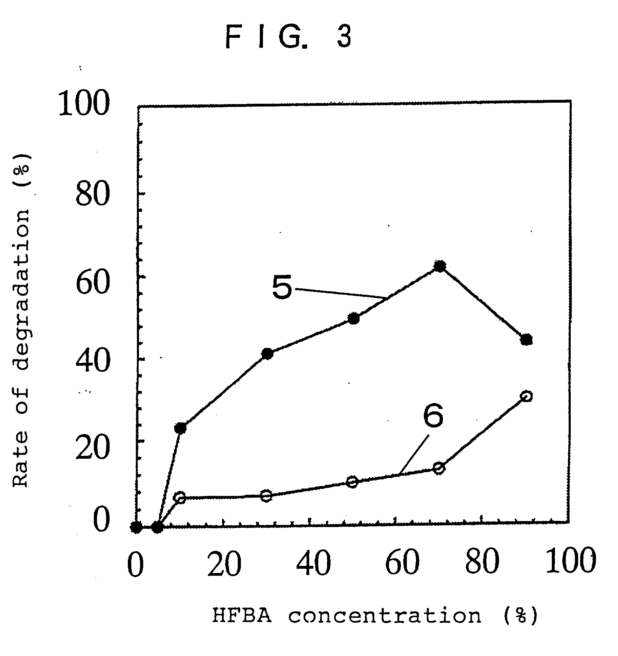

The results are shown in Fig. 3. The horizontal axis represents the concentration of HFBA. The vertical axis shows the rate of degradation of the hexadecapeptide.

Since the amino acid sequences of the proteins and peptides used in the examples of the present specification have been reported, based on the mass numbers shown by molecular ion peaks obtained by the mass spectrometry, degree of proceeded degradation can be estimated, and also products brought by the degradation can be identified. Line 5 shows the degree of degradation of the peptide bonds at the N-terminal side of serines. Line 6 shows the degree of degradation of the peptide bonds at the C-terminal side of aspartic acids.

It was shown that under these conditions, cleavage of both peptide bonds at the N-terminal side of serines and those at the C-terminal side of aspartic acids occurred when the concentration of HFBA solution exceeds 5%. Also, consecutive degradation from the C-terminus of the hexadecapeptide of Sequence No.1 used as the sample was not observed under these conditions.

### Example 3

Next, an experiment with the following reaction conditions was performed.

### (Reaction conditions)

| | |
|---|---|
| HFBA concentration | 0.01, 0.1, 0.2, 0.5, 1 and 5% |
| | (aqueous solution, containing 5% DTT) |
| Reaction temperature | 90°C |
| Reaction time | 24 hours |

Similar to Example 2, the hexadecapeptide of Sequence No.1 was used as the sample. Samples prepared according to the procedure described in Example 1 were analyzed using a mass spectrometer in the same manner as in Example 2.

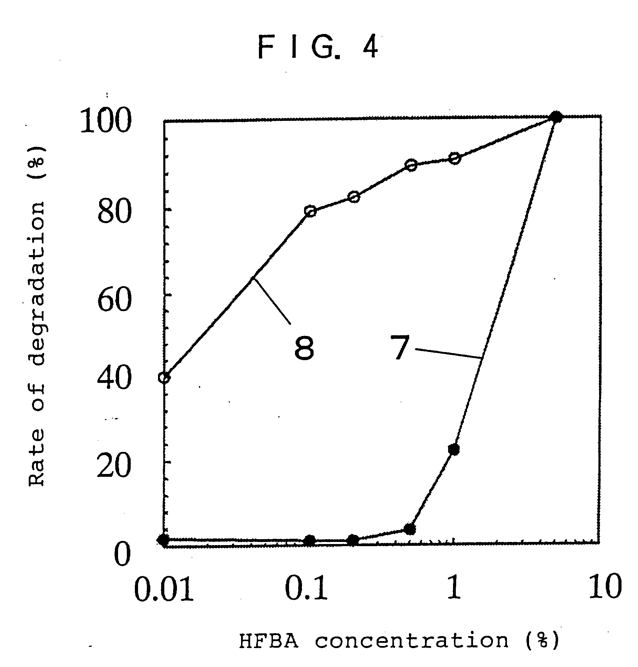

The results are shown in Fig. 4. The horizontal axis represents the concentration of HFBA. The vertical axis shows the rate of degradation of the hexadecapeptide of Sample No.1. Line 7 shows the degree of degradation of the peptide bonds at the N-terminal side of serines. Line 8 shows the degree of degradation of the peptide bonds at the C-terminal side of aspartic acids.

It was shown that under these conditions, cleavage of peptide bonds to the N-terminal side of serines occurred when the concentration of HFBA solution exceeds 0.2%. On the other hand, it was shown that under these conditions, cleavage of peptide bonds to the C-terminal side of aspartic acids occurs irrespective of the concentration of HFBA solution. Also, consecutive degradation from the C-terminus of the hexadecapeptide of Sequence No.1 used as the sample was not observed under these conditions.

### Example 4

Next, an experiment with the following reaction conditions was performed.

### (Reaction conditions)

| | |
|---|---|
| HFBA concentration | 70% |
| | (aqueous solution, containing 5% DTT) |
| Reaction temperature | 50°C |
| Reaction time | 24 hours |

Similar to Example 2, the hexadecapeptide of Sequence No.1 was used as the sample. Sample prepared according to the procedure described in Example 1 was analyzed using a mass spectrometer in the same manner as in Example 2.

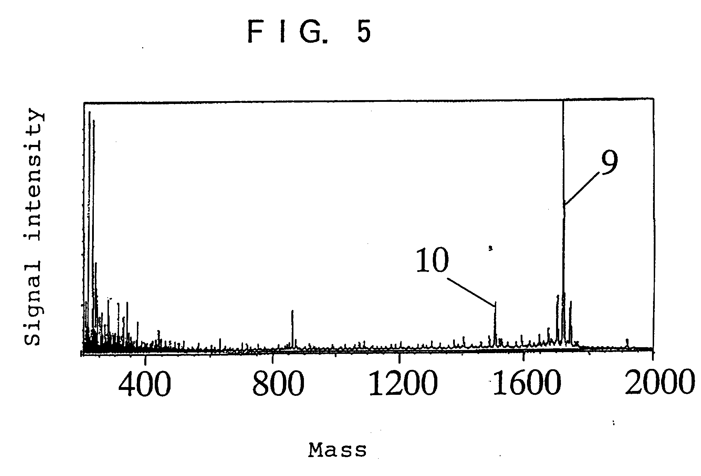

The results obtained by mass spectrometry were shown in Fig. 5. In the figure showing the results of mass spectrometry, the horizontal axis represents the mass number of each molecule, and the vertical axis represents the signal intensity.

In explaining the result of mass spectrometry, the terminology used in the present specification is as follows: When the peptide used as the sample is a hexadecapeptide, it is expressed as "peptide 1-16". Furthermore, within this hexadecapeptide, for example, a portion from the N-terminal end to the 14th amino acid residue (counting from the N-terminal) is expressed as peptide 1-14, and a portion from the 7th to the C-terminal amino acid residues is expressed as peptide 7-16. Hereunder in the present specification, the same terminology will be used. As far as special notice is not given, sequence of the amino acid residue is counted from the N-terminal end.

As was indicated by symbol 9, peptide 1-16 (the hexadecapeptide used as the sample) was detected. Also, as was indicated by symbol 10, peptide 1-14 (consisting of the N-terminal to the 14th amino acid residues of the hexadecapeptide used as the sample) was detected.

Yields of degradation (rates of degradation) were determined by comparing the height of molecular ion peaks obtained by mass spectrometry. From the determination based on the height of peaks of peptide 1-16 and peptide 1-14, the rate of degradation of peptide bond at the N-terminal side of serine (the 15th residue) was 16% under these conditions. A rate of degradation herein is obtained by dividing the height of peak of peptide 1-14 by the height of peak of peptide 1-14 plus the height of peak of peptide 1-16 then multiplying it by 100.

Under these conditions, progression of consecutive degradation from the C-terminus of the hexadecapeptide of Sequence No.1 used as the sample (for example, that is reflected by the observation in which a series of peptides consisting of consecutive number of amino acid residues such as peptides 1-16, 1-15, 1-14, 1-13, 1-12, 1-11 and 1-10 are detected at a time) was not observed.

### Example 5

Next, an experiment with the following reaction conditions was performed.

### (Reaction conditions)

| | |
|---|---|
| HFBA concentration | 75% |
| | (aqueous solution, containing 5% DTT) |
| Reaction temperature | 50°C |
| Reaction time | 24 hours |

Similar to Example 2, the hexadecapeptide of Sequence No.1 was used as the sample. Sample prepared according to the procedure described in Example 1 was analyzed using a mass spectrometer in the same manner as in Example 2.

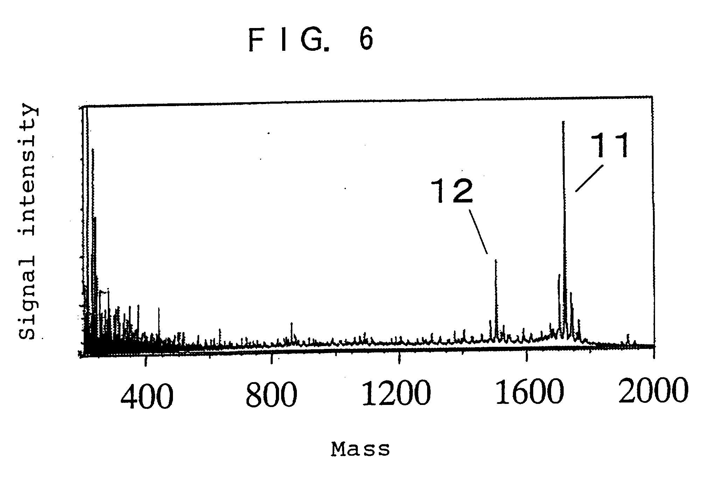

The results of the mass spectrometry are shown in Fig. 6. Expression with regard to the results of mass spectrometry and the determination of the rate of degradation are the same as described in Example 4. As indicated by symbol 11, peptide 1-16 was detected. Also, as indicated by symbol 12, peptide 1-14 was detected. Under these conditions, the rate of degradation for the peptide bond to the N-terminal side of serine (the 15th residue) was 27%.

Under these conditions, consecutive degradation from the C-terminus of the hexadecapeptide of Sequence No.1 used as the sample was not observed.

### Example 6

Next, an experiment with the following reaction conditions was performed.

### (Reaction conditions)

| | |
|---|---|
| HFBA concentration | 80% |
| | (aqueous solution, containing 5% DTT) |
| Reaction temperature | 50°C |
| Reaction time | 24 hours |

Similar to Example 2, the hexadecapeptide of Sequence No.1 was used as the sample. Sample prepared according to the procedure described in Example 1 was analyzed using a mass spectrometer in the same manner as in Example 2.

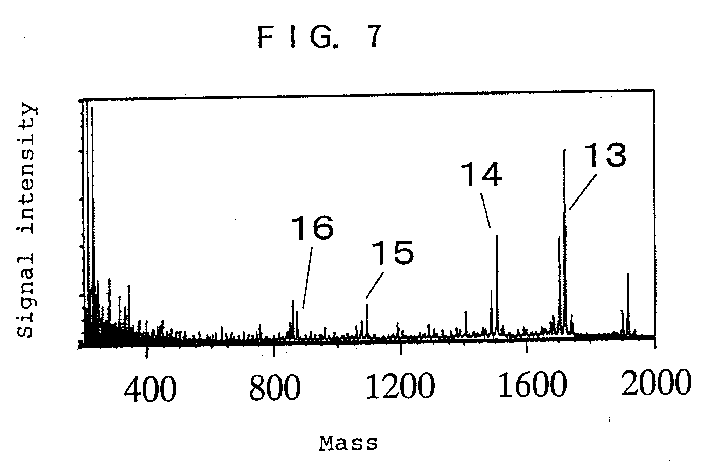

The results of the mass spectrometry are shown in Fig. 7. Expression with regard to the results of mass spectrometry and the determination of the rate of degradation are the same as described in Example 4. As indicated by symbol 13, peptide 1-16 was detected. Also, as indicated by symbol 14, peptide 1-14 was detected. Further, as indicated by symbol 15, peptide 7-16 was detected. Further as symbol 16 shows, peptide 7-14 was detected.

Under these conditions, the rate of degradation for the peptide bond at the N-terminal side of serine (the 15th residue) was 29%. Also, the rate of degradation for the peptide bond at the C-terminal side of aspartic acids (the third and sixth residues) was 17%. Under these conditions, consecutive degradation from the C-terminus of the hexadecapeptide of Sequence No.1 used as the sample was not observed.

### Example 7

Next, an experiment with the following reaction conditions was performed.

### (Reaction conditions)

| | |
|---|---|
| HFBA concentration | 85% |
| | (aqueous solution, containing 5% DTT) |
| Reaction temperature | 50°C |
| Reaction time | 24 hours |

Similar to Example 2, the hexadecapeptide of Sequence No.1 was used as the sample. Sample prepared according to the procedure described in Example 1 was analyzed using a mass spectrometer in the same manner as in Example 2.

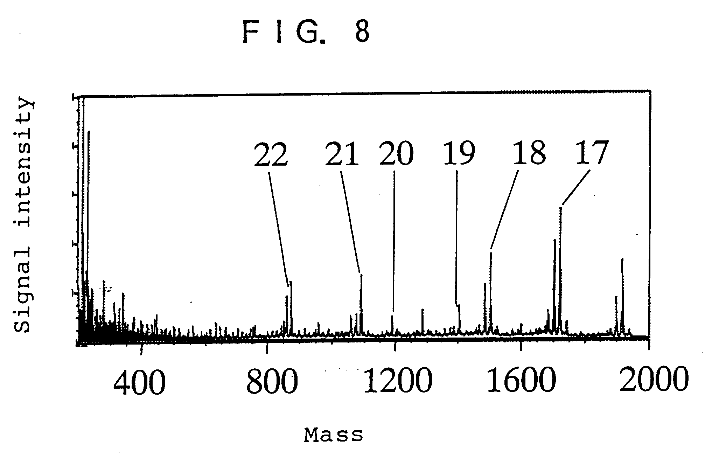

The results of the mass spectrometry are shown in Fig. 8. Expression with regard to the results of mass spectrometry and the determination of the rate of degradation are the same as described in Example 4. As indicated by symbol 17, peptide 1-16 was detected. Also, as indicated by symbol 18, peptide 1-14 was detected. Further, as indicated by symbol 19, peptide 4-16 was detected. Further, as indicated by symbol 20, peptide 4-14 was detected. Further, as indicated by symbol 21, peptide 7-16 was detected. Further, as indicated by symbol 22, peptide 7-14 was detected.

Under these conditions, the rate of degradation for the peptide bond to the N-terminal side of serine (the 15th residue) was 21%. Also, the rate of degradation for the peptide bond at the C-terminal side of aspartic acids (the third and sixth residues) was 45%.

In this case, under the conditions of Examples 4 to 7, it was difficult to monitor the degradation of peptide bond between aspartic (the 8th residue in the hexadecapeptide of Sequence No.1) and its adjacent amino acid or that between threonine (the 11th residue in the hexadecapeptide of Sequence No.1) and its adjacent amino acid, both of which have been reported to be liable to cleave under acidic conditions.

Under these conditions, consecutive degradation from the C-terminus of the C-terminal amino acid of the hexadecapeptide of Sequence No.1 used as the sample was not observed.

### Example 8

Next, an experiment with the following reaction conditions was performed.

### (Reaction conditions)

| | |
|---|---|
| HFBA concentration | 75% |
| | (aqueous solution, containing 5% DTT) |
| Reaction temperature | 50°C |
| Reaction time | 24 hours |

The decapeptide of Sequence No.2; Lys-Arg-Pro-Hyp-Gly-Phe-Ser-Pro-Phe-Arg, was used as the sample. Sample prepared according to the procedure described in Example 1 was analyzed using a mass spectrometer in the same manner as in Example 2.

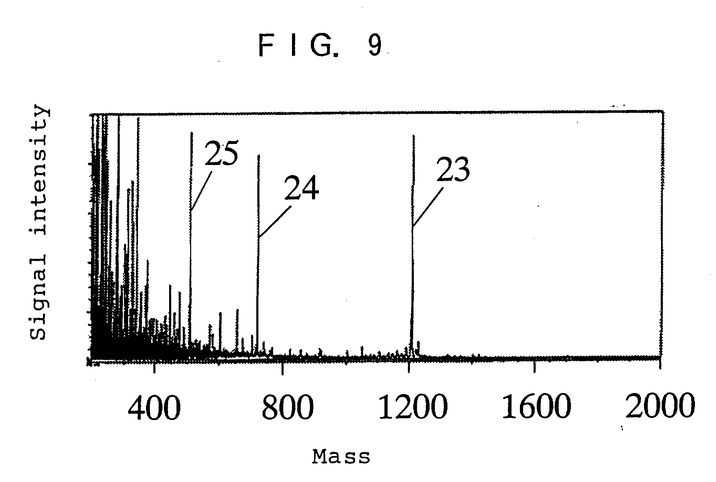

The results of the mass spectrometry were shown in Fig. 9. Expression with regard to the results of mass spectrometry and the determination of the rate of degradation are the same as described in Example 4. As indicated by symbol 23, peptide 1-10 was detected. Also, as indicated by symbol 24, peptide -6 was detected. Further, as indicated by symbol 25, peptide 7-10 was detected.

Under these conditions, the rate of degradation for the peptide bond at the N-terminal side of serine (the 7th residue) was 66%. Under these conditions, consecutive degradation from the C-terminus of the decapeptide of Sequence No.2 used as the sample was not observed. Also cleavage of other peptide bond was not observed.

### Example 9

Next, an experiment with the following reaction conditions was performed.

### (Reaction conditions)

| | |
|---|---|
| HFBA concentration | 75% |
| | (aqueous solution, containing 5% DTT) |
| Reaction temperature | 50°C |
| Reaction time | 24 hours |

The pentadecapeptide of Sequence No.3; Lys-Ile-Leu-Gly-Asn-Gln-Gly-Ser-Phe-Leu-Thr-Lys-Gly-Pro-Ser-Lys-Leu, was used as the sample. Samples prepared according to the procedure described in Example 1 was analyzed using a mass spectrometer in the same manner as in Example 2.

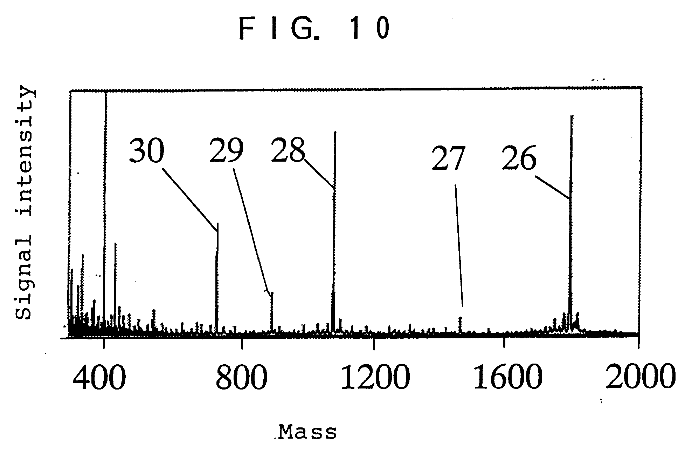

The results of the mass spectrometry were shown in Fig. 10. Expression with regard to the results of mass spectrometry and the determination of the rate of degradation are the same as described in Example 4. As indicated by symbol 26, peptide 1-17 was detected. Also, as indicated by symbol 27, peptide 1-14 was detected. Further, as indicated by symbol 28, peptide 8-17 was detected. Further, as indicated by symbol 29, peptide 1-17 was detected. This molecular ion was divalently charged. Further, as was indicated by symbol 30, peptide 1-7 was detected.

Under these conditions, consecutive degradation from the C-terminus of the pentadecapeptide of Sequence No.3 used as the sample was not observed. Under these conditions, regarding the two serines included in the pentadecapeptide of Sequence No.3, the rate of degradation for the peptide bond to the N-terminal side of the 8th serine was 58% and that of the 15th serine was 3%.

Further, degradation of peptide bonds between glycines (the 4th and 13th residues in the pentadecapeptide of Sequence No.3) and their adjacent amino acids, those between aspartic acid(the 5th residue in the pentadecapeptide of Sequence No.3) and its adjacent amino acids and those between threonine (the 11th residue in the pentadecapeptide of Sequence No.3) and its adjacent amino acids, either of which has been reported to be liable to cleave under an acidic conditions, was not observed.

### Example 10

Next, an experiment with the following reaction conditions was performed.

### (Reaction conditions)

| | |
|---|---|
| HFBA concentration | 75% |
| | (aqueous solution, containing 5% DTT) |
| Reaction temperature | 50°C |
| Reaction time | 24 hours |

The undecapeptide of Sequence No.4; Glp-Lys-Arg-Pro-Ser-Gln-Arg-Ser-Lys-Tyr-Leu, was used as the sample. Sample prepared according to the procedure described in Example 1 was analyzed using a mass spectrometer in the same manner as in Example 2.

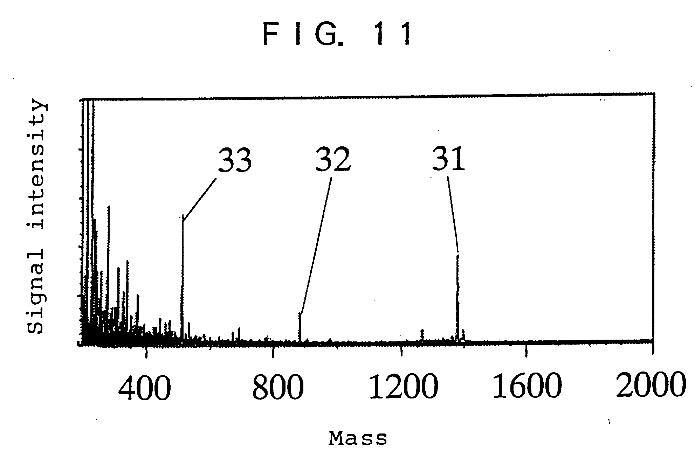

The results of the mass spectrometry were shown in Fig. 11. Expression with regard to the results of mass spectrometry and the determination of the rate of degradation are the same as described in Example 4. As indicated by symbol 31, peptide 1-11 was detected. Also, as indicated by symbol 32, peptide 1-7 was detected. Further, as indicated by symbol 33, peptide8-11 was detected.

Under these conditions, consecutive degradation from the C-terminus of the undecapeptide of Sequence No.4 used as the sample was not observed. Under these conditions, regarding the two serines included in the undecapeptide of Sequence No.4, the rate of degradation for the peptide bond to the N-terminal side of the 8th serine was 65%. Almost no cleavage of the peptide bond 'at the N-terminal side of the 5th serine was observed.

### Example 11

Next, an experiment with the following reaction conditions was performed.

### (Reaction conditions)

| | |
|---|---|
| HFBA concentration | 75% |
| | (aqueous solution, containing 5% DTT) |
| Reaction temperature | 50°C |
| Reaction time | 24 hours |

As samples, alcohol dehydrogenase obtained from yeast (ADH), cucumber green mottle mosaic virus coat protein (CGMMV), tobacco mosaic virus coat protein (TMV), and snake venom protein TM-1 obtained from Taiwan habu (TM-1) were used. Samples prepared according to the procedure described in Example 1 were analyzed using sodium dodecylsulfate-Tricine-polyacrylamide gel electrophoresis (SDS/Tricine/PAGE) . Conditions and procedures for SDS/Tricine/PAGE are well known, and therefore detailed description thereof is omitted.

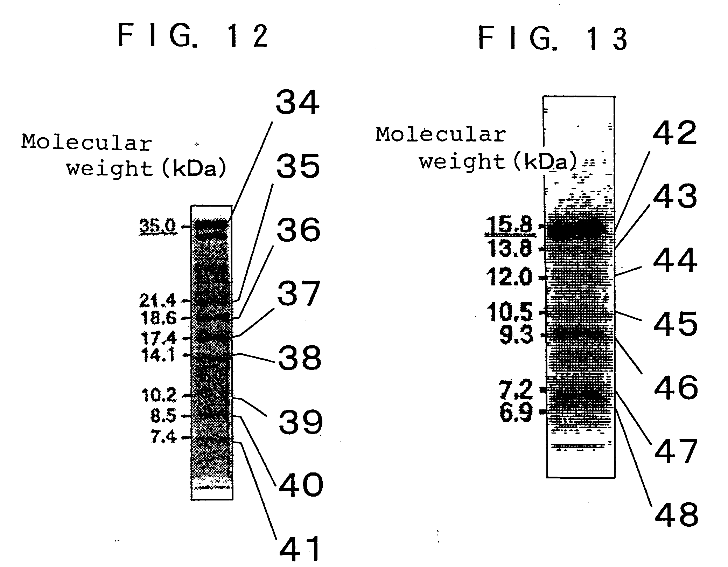

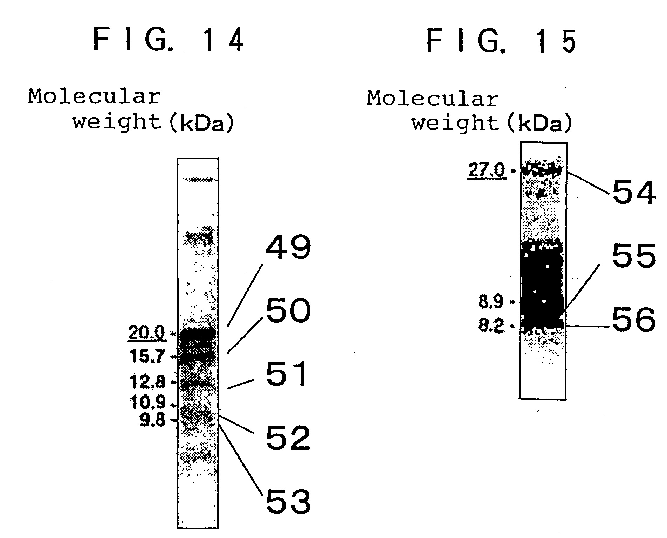

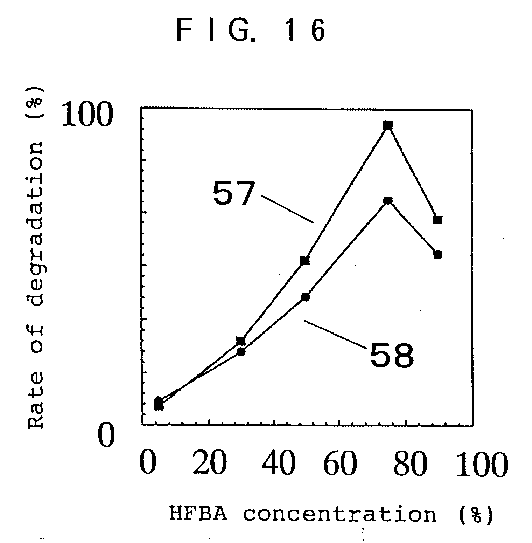

Fig. 12 shows the results of analysis of ADH (molecular weight; 35 kDa) . Fig. 13 shows the results of analysis of CGMMV (molecular weight; 15.8 kDa). Fig. 14 shows the results of analysis of TMV (molecular weight; 20 kDa). Fig. 15 shows the results of analysis of TM-1 (molecular weight; 27 kDa).

In any case of either sample, more than one peptide bands were observed, which show that specific degradation of proteins was proceeded.

Next, N-terminal amino acid sequencing of obtained peptides was performed. Procedures used for this analysis includes two processes, electroblotting of peptides isolated by electrophoresis onto polyvinylidenefluoride membranes and amino acid sequencing using a protein sequencer. These two processes are both well known, and therefore, the detailed description thereof is omitted.

### (Results of cleavage of ADH)

ADH is a protein consisting of 347 amino acids with an acetylated amino acid at the N-terminus , and its total amino acid sequence has been reported.

A band corresponding to ADH was detected. (symbol 34)

The N-terminal amino acid sequence of a peptide obtained from the band of molecular weight of 21.4 kDa (symbol 35) was Thr-Asp-Leu-Ala-Gln-. Based on this sequence and apparent molecular weight, it was proved that the peptide bond at the N-terminal side of the 143th threonine was cleaved.

The N-terminal amino acid sequence of a peptide obtained from the band of molecular weight of 18.6 kDa (symbol 36) was Ser-Leu-Ala-Val-Gln-. Based on this sequence and apparent molecular weight, it was proved that the peptide bond at the N-terminal side of the 184th serine was cleaved.

The N-terminal amino acid sequence of a peptide obtained from the band of molecular weight of 17.4 kDa (symbol 37) was Ser-Ile-Pro-Glu-Thr-. Based on this sequence and apparent molecular weight, it was proved that the acetyl group of the N-terminal serine and the peptide bond at the N-terminal side of the 164th serine were cleaved.

The N-terminal amino acid sequence of a peptide obtained from the band of molecular weight of 14.1 kDa (symbol 38) was Ser-Ile-Pro-Glu-Thr-. Based on this sequence and apparent molecular weight, it was proved that the acetyl group of the N-terminal serine and the peptide bond at the N-terminal side of the 124th serine were cleaved.

The N-terminal amino acid sequence of a peptide obtained from the band of molecular weight of 10.2 kDa (symbol 39) was Ser-Ile-Pro-Glu-Thr-. Based on this sequence and apparent molecular weight, it was proved that the acetyl group of the N-terminal serine and the peptide bond at the N-terminal side of the 96th serine were cleaved.

The N-terminal amino acid sequence of a peptide obtained from the band of molecular weight of 8.5 kDa (symbol 40) was Thr-Thr-Val-Leu-Val-. Based on this sequence and apparent molecular weight, it was proved that the peptide bond at the N-terminal side of the 264th threonine was cleaved.

The N-terminal amino acid sequence of a peptide obtained from the band of molecular weight of 7.4 kDa (symbol 41) was Ser-Asp-Val-Phe-Asn-. Based on this sequence and apparent molecular weight, it was proved that the peptide bond at the N-terminal side of the 278th serine was cleaved.

### (Results of cleavage of CCMMV)

CCMMV is a protein consisting of 160 amino acids with an acetylated amino acid at the N-terminal, and its total amino acid sequence has been reported.

A band corresponding to CCMMV was detected. (symbol 42)

The N-terminal amino acid sequence of a peptide obtained from the band of molecular weight of 13.8 kDa (symbol 43) was Ser-Tyr-Val-Pro-Val-. Based on this sequence and apparent molecular weight, it was proved that peptide bonds at the N-terminal side of the 16th and 143th serines were cleaved.

The N-terminal amino acid sequence of a peptide obtained from the band of molecular weight of 12.0 kDa (symbol 44) was Thr-Ala-Phe-Gln-Thr-. Based on this sequence and apparent molecular weight, it was proved that the peptide bond at the N-terminal side of the 33th and that at the N-terminal side of the 143th serine were cleaved.

The N-terminal amino acid sequence of a peptide obtained from the band of molecular weight of 10.5 kDa (symbol 45) was Ser-Ala-Leu-Pro-Ser-. Based on this sequence and apparent molecular weight, it was proved that the peptide bond at the N-terminal side of the 49th and that at the N-terminal side of the 143th serines were cleaved.

The N-terminal amino acid sequence of a peptide obtained from the band of molecular weight of 9.3 kDa (symbol 46) was Ser-Arg-Phe-Pro-Asp-. Based on this sequence and apparent molecular weight, it was proved that the peptide bond at the N-terminal side of the 60th and that to the N-terminal side of 143th serines were cleaved.

The N-terminal amino acid sequence of a peptide obtained from the band of molecular weight of 7.2 kDa (symbol 47) was Pro-Ser-Asn-Pro-Thr-. Based on this sequence and apparent molecular weight, it was proved that the peptide bond at the N-terminal side of the 99th proline was cleaved.

The N-terminal amino acid sequence of a peptide obtained from the band of molecular weight of 6.9 kDa (symbol 48) was Pro-Ser-Asn-Pro-Thr-. Based on this sequence and apparent molecular weight, it was proved that the peptide bond at the N-terminal side of the 99th proline and that at the N-terminal side of the 158th serine were cleaved.

### (Results of cleavage of TMV)

TMV is a protein consisting of 158 amino acids and with an acetylated amino acid at the N-terminal, and its total amino acid sequence has been reported.

A band corresponding to TMV was detected. (symbol 49)

The N-terminal amino acid sequence of a peptide obtained from the band of molecular weight of 15.7 kDa (symbol 50) was Ser-Tyr-Ser-Ile-Thr-. Based on this sequence and apparent molecular weight, it was proved that the acetyl group of the N-terminal end and the peptide bond at the N-terminal side of the 136th threonine were cleaved.

The N-terminal amino acid sequence of a peptide obtained from the band of molecular weight of 12.8 kDa (symbol 51) was Ser-Glu-Val-Trp-Lys-. Based on this sequence and apparent molecular weight, it was proved that the peptide bond at the N-terminal side of the 49th serine was cleaved.

The N-terminal amino acid sequence of a peptide obtained from the band of molecular weight of 10.9 kDa (symbol 52) was Ser-Asp-Phe-Lys-Val-. Based on this sequence and apparent molecular weight, it was proved that the peptide bond at the N-terminal side of the 65th serine was cleaved.

The N-terminal amino acid sequence of a peptide obtained from the band of molecular weight of 9.8 kDa (symbol 53) was Pro-Leu-Val-Thr-Ala-. Based on this sequence and apparent molecular weight, it was proved that the peptide bond at the N-terminal side of the 78th proline was cleaved.

### (Results of cleavage of TM-1)

A band corresponding to TM-1 was detected (symbol 54). TM-1 is a protein consisting of 203 amino acids, and its total amino acid sequence has been reported.

The N-terminal amino acid sequence of a peptide obtained from the band of molecular weight of 8.9 kDa (symbol 55) was Ser-Val-Gly-Leu-Val-. Based on this sequence and apparent molecular weight, it was proved that the peptide bond at the N-terminal side of the 124th serine was cleaved.

The N-terminal amino acid sequence of a peptide obtained from the band of molecular weight of 8.2 kDa (symbol 56) was Ser-Ser-Asn-Val-Phe-. Based on this sequence and apparent molecular weight, it was proved that the peptide bond at the N-terminal side of the 132th serine was cleaved.

Thus, it was proved that peptide bonds at the N-terminal side of serine were cleaved under conditions used in the present example. In addition, cleavage of acetyl group of N-terminal acetylated serine, cleavage of aspartylprolyl bond, and cleavage of glycylthreonyl bond were observed.

### Example 12

Under the conditions used in Example 11, cleavage of glycylthreonyl bond was observed. On the other hand, peptide bond at the N-terminal side of threonine was not cleaved in the following portion that is present within in ADH, CGMMV, TMV or TM-1 used as samples.

The following respective bonds were not cleaved; alanylthreonine, aspartylthreonine, serylthreonine, glutamoylthreonine, histidylthreonine, isoleucylthreonine, phenylalanylthreonine, threonylthreonine, tyrosylthreonine, arginylthreonine, glutamylthreonine, prolylthreonine, valylthreonine, cysteinylthreonine, tryptophanylthreonine, leucylthreonine, asparaginylthreonine and methionylthreonine bonds.

Next, an experiment with the following reaction conditions was performed.

### (Reaction conditions)

| | |
|---|---|
| HFBA concentration | 5, 30, 50, 75 and 90% |
| | (aqueous solution, containing 5% DTT) |
| Reaction temperature | 50°C |
| Reaction time | 24 hours |

The results are listed in Fig. 16.

As samples, two dipeptides, glycylthreonine and glycylserine, were used. The rate of degradation was determined based on the results of amino acid analysis. Procedure for amino acid analysis is well known and therefore is not mentioned here. Line 57 shows the degradation rates of glycylthreonine. Line 58 shows the degradation rates of glycylserine.

It was shown that under these conditions, except that of 5% HFBA concentration, glycylthreonyl bond was more liable to cleave compared to glycylseryl bond.

### Example 13

Next, an experiment with the following reaction conditions was performed.

### (Reaction conditions)

| | |
|---|---|
| HFBA concentration | 90% |
| | (aqueous solution, containing 5% DTT) |
| Reaction temperature | 30°C |
| Reaction time | 24 hours |

ADH was used as a sample. Sample prepared according to the procedure described in Example 1 was analyzed using sodium dodecylsulfate-Tricine-polyacrylamide gel electrophoresis (SDS/Tricine/PAGE), in the same manner as in Example 11.

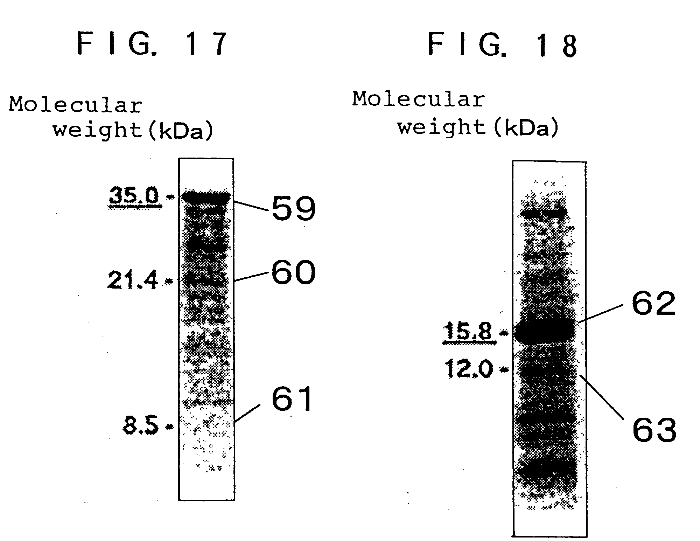

The obtained results are shown in Fig. 17. Further, in the same manner as in Example 11, N-terminal amino acid sequencing of isolated peptides was performed. A band corresponding to ADH was detected (symbol 59). The results on each peptide obtained from the band of molecular weight of 21.4 kDa (symbol 60) and that of molecular weight of 8.5 kDa (symbol 61), respectively, are the same with those obtained in Example 11.

It was shown that under these conditions, glycylthreonyl bond was cleaved.

### Example 14

Next, an experiment with the following reaction conditions was performed.

### (Reaction conditions)

| | |
|---|---|
| HFBA concentration | 5% |
| | (aqueous solution, containing 5% DTT) |
| Reaction temperature | 40°C |
| Reaction time | 24 hours |

CGMMV was used as a sample. Sample prepared according to the procedure described in Example 1 was analyzed using sodium dodecylsulfate-Tricine-polyacrylamide gel electrophoresis (SDS/Tricine/PAGE), in the same manner as Example 11.

The obtained results were shown in Fig. 18. Further, in the same manner as in Example 11, N-terminal amino acid sequencing of isolated peptides was performed. A band corresponding to CGMMV was detected (symbol 62). The results on a peptide obtained from the band of molecular weight of 12.0 kDa (symbol 63) are the same with that obtained in Example 11. It was proved that under these conditions, glycylthreonyl bond was cleaved.

### Example 15

Next, an experiment with the following reaction conditions was performed.

### (Reaction conditions)

| | |
|---|---|
| HFBA concentration | 75% |
| | (aqueous solution, containing 5% DTT) |
| Reaction temperature | 30°C |
| Reaction time | 24 hours |

TMV was used as a sample. Sample prepared according to the procedure described in Example 1 was analyzed using sodium dodecylsulfate-Tricine-polyacrylamide gel electrophoresis (SDS/Tricine/PAGE), in the same manner as in Example 11.

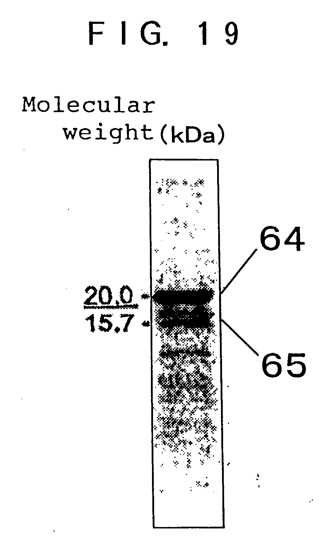

The obtained results were shown in Fig. 19. Further, in the same manner as in Example 11, N-terminal amino acid sequencing of isolated peptides was performed. A band corresponding to TMV was detected (symbol 64) . The results on a peptide obtained from the band of molecular weight of 15.7 kDa (symbol 65) are the same with that obtained in Example 11.

It was proved that under these conditions, glycylthreonyl bond was cleaved.

### Example 16

Next, an experiment with the following reaction conditions was performed.

### (Reaction conditions)

| | |
|---|---|
| HFBA concentration | 0.2% |
| | (aqueous solution, containing 5% DTT) |
| Reaction temperature | 90°C |
| Reaction time | 4 hours |

Similar to Example 2, the hexadecapeptide of Sequence No.1 was used as a sample. Sample prepared according to the procedure described in Example 1 was analyzed using a mass spectrometer in the same manner as in Example 2.

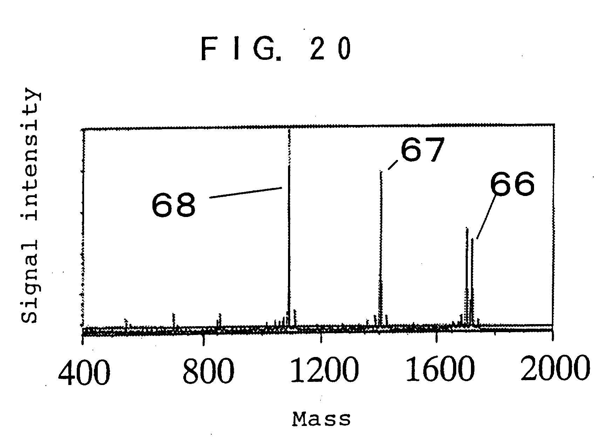

The results of the mass spectrometry were shown in Fig. 20. Expression with regard to the results of mass spectrometry and the determination of the rate of degradation are the same as described in Example 4. As was indicated by symbol 66, peptide 1-16 was detected. Peptide 4-16 that is obtained through cleavage of the peptide bond at the C-terminal side of third aspartic acid was observed (symbol 67). Further, peptide 7-16 which is obtained through cleavage of the peptide bond at the C-terminal side of the 6th aspartic acid was observed (symbol 68).

The rate of degradation for peptide bond at the C-terminal side of aspartic acid was approximately 65%. Under these conditions, consecutive degradation from the C-terminus of the hexadecapeptide of Sequence No.1 used as the sample was not observed.

### Example 17

Next, an experiment with the following reaction conditions was performed.

### (Reaction conditions)

| | |
|---|---|
| HFBA concentration | 0.2% |
| | (aqueous solution, containing 5% DTT) |
| Reaction temperature | 90°C |
| Reaction time | 4 hours |

As a sample, the tridecapeptide of Sequence No.5; Arg-Arg-Leu-Ile-Glu-Asp-Ala-Glu-Tyr-Ala-Ala-Arg-Gly, was used.

Sample prepared according to the procedure described in Example 1 was analyzed using a mass spectrometer in the same manner as in Example 2.

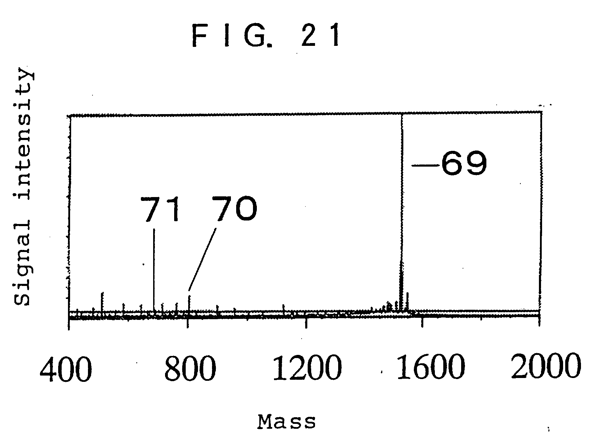

The results of the mass spectrometry were shown in Fig. 21. Expression with regard to the results of mass spectrometry and the determination of the rate of degradation are the same as described in Example 4. As was indicated by symbol 69, peptide 1-13 was detected. Peptide 1-6 that generates through cleavage of the peptide bond at the C-terminal side of 6th aspartic acid was observed (symbol 70).

Further, peptide 1-5 that is obtained through cleavage of the peptide bond at the N-terminal side of the 6th aspartic acid was observed (symbol 71). The rate of degradation for the peptide bond at the C-terminal side of aspartic acid was approximately 9%. The rate of degradation for the peptide bond at the N-terminal side of aspartic acid was approximately 2%.

Under these conditions, consecutive degradation from the C-terminus of the tridecapeptide of Sequence No.5 used as the sample was not observed.

### Example 18

Next, an experiment with the following reaction conditions was performed.

### (Reaction conditions)

| | |
|---|---|
| HFBA concentration | 0.2% |
| | (aqueous solution, containing 5% DTT) |
| Reaction temperature | 90°C |
| Reaction time | 8 hours |

As a sample, the tridecapeptide of Sequence No.5 was used as well as in Example 17.

Sample prepared according to the procedure described in Example 1 was analyzed using a mass spectrometer in the same manner as in Example 2.

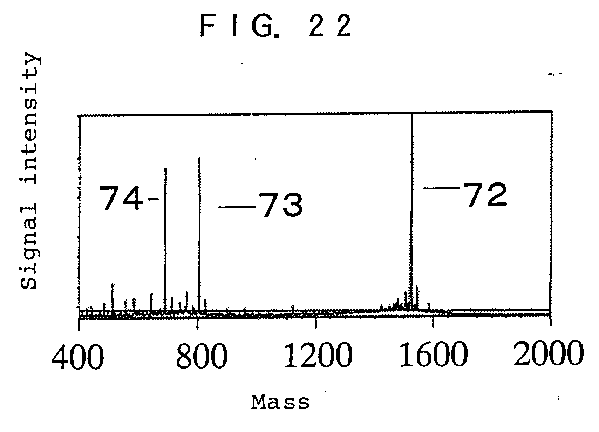

The results of the mass spectrometry were shown in Fig. 22. Expression with regard to the results of mass spectrometry and the determination of the rate of degradation are the same as described in Example 4. As shown by symbol 72, peptide 1-13 was detected. Peptide 1-6 that is obtained through cleavage of the peptide bond at the C-terminal side of 6th aspartic acid was observed (symbol 73). Further, peptide 1-5 that is obtained through cleavage of the peptide bond at the N-terminal side of the 6th aspartic acid was observed (symbol 74). The rate of degradation for the peptide bond at the C-terminal side of aspartic acid was approximately 31%. The rate of degradation for the peptide bond at the N-terminal side of aspartic acid was approximately 29%.

Under these conditions, consecutive degradation from the C-terminus of the tridecapeptide of Sequence No.5 used as the sample was not observed.

### Example 19

Next, an experiment with the following reaction conditions was performed.

### (Reaction conditions)

| | |
|---|---|
| HFBA concentration | 0.2% |
| | (aqueous solution, containing 5% DTT) |
| Reaction temperature | 90°C |
| Reaction time | 24 hours |

As a sample, the tridecapeptide of Sequence No.5 was used as well as in Example 17.

Sample prepared according to the procedure described in Example 1 was analyzed using a mass spectrometer in the same manner as in Example 2.

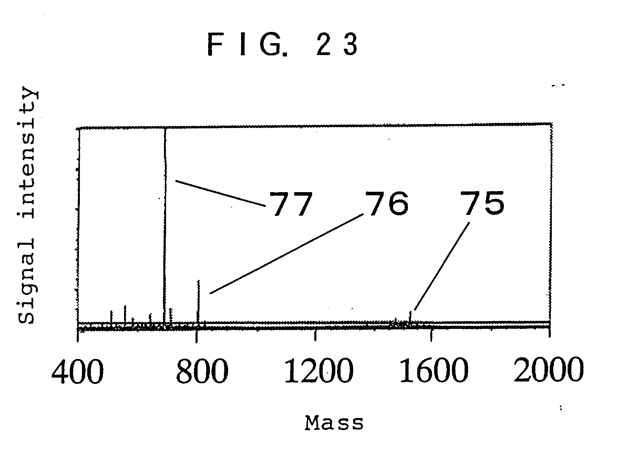

The results of the mass spectrometry were shown in Fig. 23. Expression with regard to the results of mass spectrometry and the determination of the rate of degradation are the same as described in Example 4. As shown by symbol 75, peptide 1-13 was detected. Peptide 1-6 that is obtained through cleavage of the peptide bond at the C-terminal side of 6th aspartic acid was observed (symbol 76). Further, peptide 1-5 which is obtained through cleavage of the peptide bond at the N-terminal side of 6th aspartic acid was observed (symbol 77). The rate of degradation for the peptide bond at the C-terminal side of aspartic acid was approximately 18%. The rate of degradation for the peptide bond at the N-terminal side of aspartic acid was approximately 77%.

Under these conditions, consective degradation from the C-terminus of the tridecapeptide of Sequence No.5 used as the sample was not observed.

From a series of experiments from Experiment 17 to Experiment 19, it was proved that under these conditions, first, cleavage of peptide bonds at the C-terminal side of aspartic acid residues proceeded, and then cleavage of peptide bonds at the N-terminal side of aspartic acid residues proceeded.

### Example 20

Next, an experiment with the following reaction conditions was performed.

### (Reaction conditions)

| | |
|---|---|
| HFBA concentration | 0.2% |
| | (aqueous solution, containing 5% DTT) |
| Reaction temperature | 90°C |
| Reaction time | 4 hours (8 hours for H2A) |

As samples, cytochrome c, myoglobin obtained from cormorant (myoglobin), TM-1 and histon H2A protein (H2A) were used.

Samples prepared according to the procedure described in Example 1 were analyzed using sodium dodecylsulfate-Tricine-polyacrylamide gel electrophoresis (SDS/Tricine/PAGE), in the same manner as in Example 11.

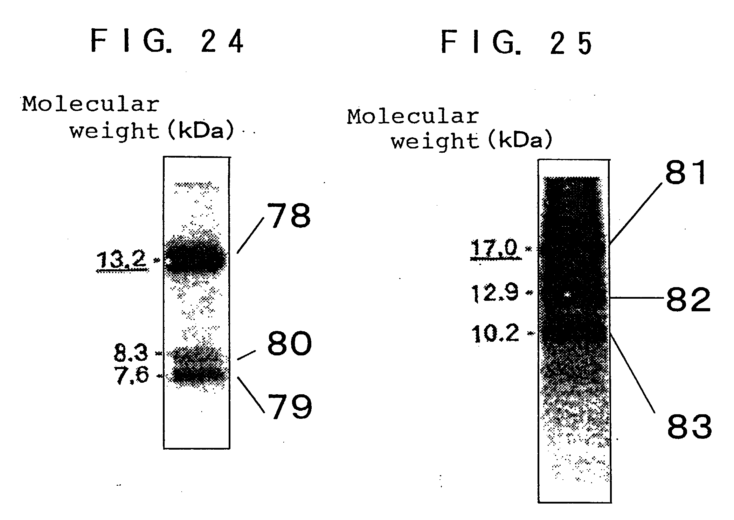

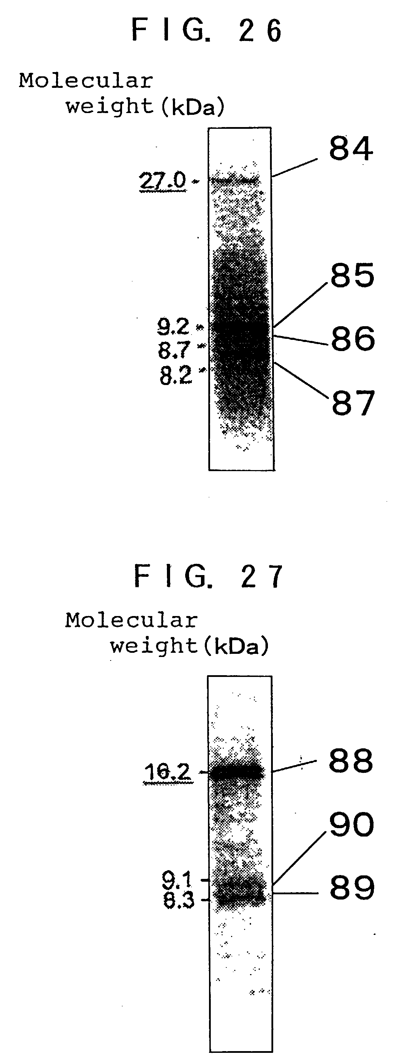

Fig. 24 shows the results of analysis of cytochrome c (molecular weight:13.2 kDa). Fig. 25 shows the results of analysis of myoglobin (molecular weight:17.0 kDa). Fig. 26 shows the results of analysis of TM-1 (molecular weight:27.0 kDa). Fig. 26 shows the results of analysis of H2A (molecular weight:16.2 kDa).

Further, in the same manner as in Example 11, N-terminal amino acid sequencing of isolated peptides was performed.

### (Results of degradation cleavage of cytochrome c)

Cytochrome c is a protein consisting of 104 amino acids with an acetylated amino acid at the N-terminus , and its total amino acid sequence has been reported.

A band corresponding to cytochrome c was detected.

### (symbol 78)

The N-terminal amino acid sequence of a peptide obtained from the band of molecular weight of 7.6 kDa (symbol 79) was Ala-Asn-Lys-Asn-lys-. Base on this sequence and apparent molecular weight, it was proved that the peptide bond at the C-terminal side of the 50th aspartic acid was cleaved.

The N-terminal amino acid sequence of a peptide obtained from the band of molecular weight of 8.3 kDa (symbol 80) could not be obtained. From the fact, it was suggested that the peptide bond at the C-terminal side of the 50th aspartic acid was cleaved.

### (Results of cleavage of myoglobin)

Myoglobin is a protein consisting of 153 amino acids and its total amino acid sequence has been reported.

A band corresponding to myoglobin was detected. (symbol 81)

The N-terminal amino acid sequences of peptides obtained from the band of molecular weight of 12.9 kDa (symbol 82) were His-Pro-Glu-Thr-leu- and Arg-Phe-Glu-Arg-Phe-. Based on these sequences and apparent molecular weight, it was proved that peptide bonds at the C-terminal side of the 35th and 41st aspartic acids were cleaved, respectively.

The N-terminal amino acid sequence of a peptide obtained from the band of molecular weight of 10.2 kDa (symbol 83) was Leu-Lys-Lys-His-Gly-. Based on this sequence and apparent molecular weight, it was proved that the peptide bond at the C-terminal side of the 60th aspartic acid was cleaved.

### (Results of cleavage of TM-1)

TM-1 is a protein consisting of 203 amino acids and its total amino acid sequence has been reported.

A band corresponding to TM-1 was detected. (symbol 84)

The N-terminal amino acid sequence of peptide obtained from the band of molecular weight of 9.2 kDa (symbol 85) was Pro-Lys-Phe-Ser-Val-. Based on this sequence and apparent molecular weight, it was proved that peptide bond at the C-terminal side of the 120th aspartic acid was cleaved.

The N-terminal amino acid sequence of a peptide obtained from the band of molecular weight of 8.7 kDa (symbol 86) was Pro-Lys-Phe-Ser-Val-. Based on this sequence and apparent molecular weight, it was proved that the peptide bonds at the C-terminal side of the 120th and 194th aspartic acids were cleaved.

The N-terminal amino acid sequence of a peptide obtained from the band of molecular weight of 8.2 kDa (symbol 87) was Met-Try-Lys-Pro-Leu-. Based on this sequence and apparent molecular weight, it was proved that the peptide bonds at the C-terminal side of the 44th and 120th aspartic acids were cleaved.

### (Results of cleavage of H2A)

H2A is a protein consisting of 129 amino acids with an acetylated amino acid at the N-terminal, and its e total amino acid sequence has been reported.

A band corresponding to H2A was detected. (symbol 88)

The N-terminal amino acid sequence of peptide obtained from the band of molecular weight of 8.3 kDa (symbol 89) was Asn-Lys-Lys-Thr-Arg-. Based on this sequence and apparent molecular weight, it was proved that the peptide bond at the C-terminal side of the 72nd aspartic acid was cleaved.

The N-terminal amino acid sequence of a peptide obtained from the band of molecular weight of 9.1 kDa (symbol 90) could not be obtained. From the fact, it was suggested that the peptide bond at the C-terminal side of the 92nd aspartic acid was cleaved.

Thus, it is proved that under the conditions of the present examples, peptide bonds at the C-terminal side of aspartic acid residues can be cleaved.

### Example 21

Next, an experiment with the following reaction conditions was performed.

### (Reaction conditions)

| | |
|---|---|
| HFBA concentration | 0.2% |
| | (aqueous solution, containing 5% DTT) |
| Reaction temperature | 60°C |
| Reaction time | 16 hours |

As a sample, the dodecapeptide of Sequence No.6: Val-Ser-Ser-Asn-Ile-Ser-Glu-Asp-Pro-Val-Pro-Val, was used. Sample prepared according to the procedure described in Example 1 was analyzed using a mass spectrometer in the same manner as in Example 2.

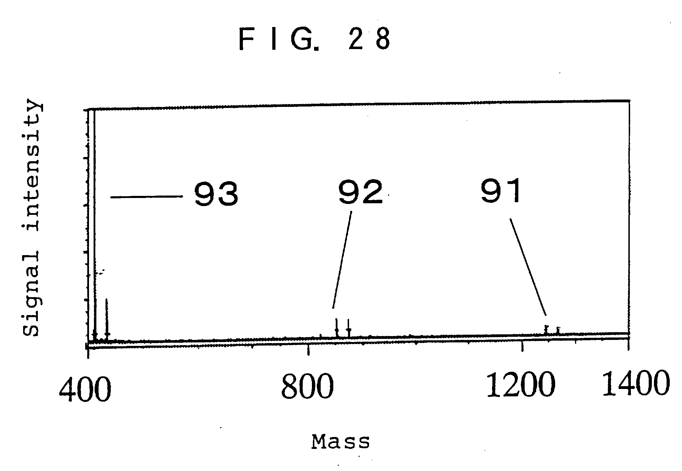

The results of the mass spectrometry were shown in Fig. 28. Expression regarding the results of mass spectrometry is the same as described in Example 4. As was indicated by symbol 91, peptide 1-12 was detected. Further, as was indicated by symbol 92, peptide 1-8 was detected. Further, as was indicated by symbol 93, peptide 9-12 was detected.

Under these conditions, consecutive degradation from the C-terminus of the dodecapeptide of Sequence No.6 used as the sample was not observed. Based on the fact that peptide 1-8 and peptide 9-12 were observed, it was proved that aspartylprolyl bond was cleaved.

### Example 22

Next, an experiment with the following reaction conditions was performed, in the same manner as Example 21.

### (Reaction conditions)

| | |
|---|---|
| HFBA concentration | 0.2% |
| | (aqueous solution, containing 5% DTT) |
| Reaction temperature | 60°C |
| Reaction time | 16 hours |

As a sample, the tridecapeptide of Sequence No.5 was used. Sample prepared according to the procedure described in Example 1 was analyzed using a mass spectrometer in the same manner as in Example 2.

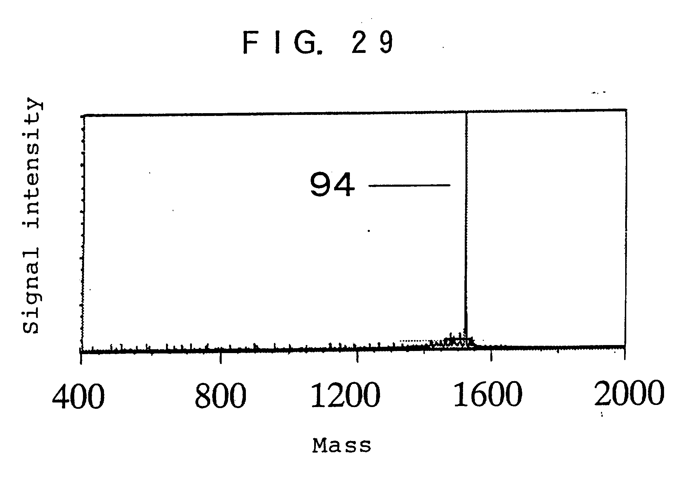

The results of the mass spectrometry were shown in Fig. 29. Expression regarding the results of mass spectrometry is the same as in Example 4. As was indicated by the symbol 94, peptide 1-13 was detected.

Under these conditions, consecutive degradation from the C-terminus of the tridecapeptide of Sequence No.5 used as the sample was not observed. Only signal originated from the tridecapeptide of Sequence No.5 used as the sample was detected, and it was proved that under the reaction conditions of the present example, the aspartylalanyl bond was not cleaved.

### Example 23

Next, an experiment with the following reaction conditions was performed, in the same manner as in Example 21.

### (Reaction conditions)

| | |
|---|---|
| HFBA concentration | 0.2% |
| | (aqueous solution, containing 5% DTT) |
| Reaction temperature | 60°C |
| Reaction time | 16 hours |

As samples, carboxylic acid dehydrogenase II (CA-II) and CGMMV were used.

Samples prepared according to the procedure described in Example 1 were analyzed using sodium dodecylsulfate-Tricine-polyacrylamide gel electrophoresis (SDS/Tricine/PAGE), in the same manner as in Example 11.

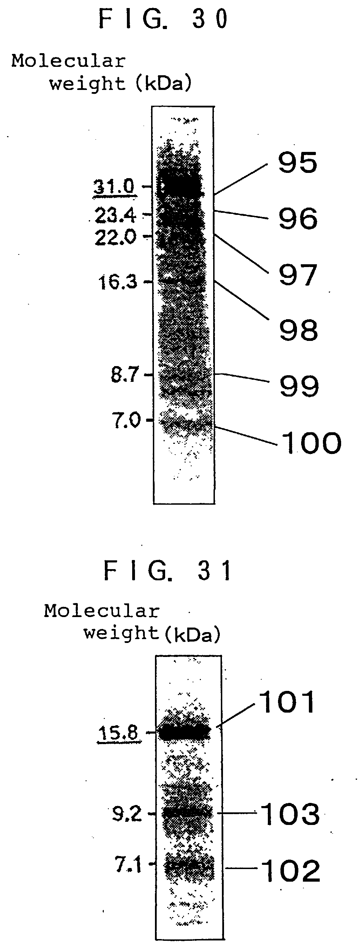

Fig. 30 shows the results of analysis of CA-II (molecular weight:31.0 kDa). Fig. 31 shows the results of analysis of CGMMV (molecular weight:15.8 kDa).

Further, in the same manner as in Example 11, N-terminal amino acid sequencing of isolated peptides was performed.

### (Results of degradation cleavage of CA-II)

CA-II is a protein consisting of 259 amino acids with acetylated N-terminal amino acid residue, and its total amino acid sequence has been reported.

A band corresponding to CA-II was detected. (symbol 95)

The N-terminal amino acid sequence of a peptide obtained from the band of molecular weight of 23.4 kDa (symbol 96) was Pro-Ala-Leu-Lys-Pro-. Based on this sequence and apparent molecular weight, it was proved that the peptide bond at the C-terminal side of the 40th aspartic acid (aspartylprolyl bond) was cleaved.

The N-terminal amino acid sequence of a peptide obtained from the band of molecular weight of 22.0 kDa (symbol 97) could not be obtained. From the fact, it was suggested that the peptide bond at the C-terminal side of the 178th aspartic acid (aspartylprolyl bonds) was cleaved.

The N-terminal amino acid sequence of a peptide obtained from the band of molecular weight of 13.6 kDa (symbol 98) was Pro-Ala-Leu-Lys-Pro-. Based on this sequence and apparent molecular weight, it was proved that peptide bonds at the C-terminal side of the 40th and 178th aspartic acids (aspartylprolyl bonds) were cleaved, respectively.

The N-terminal amino acid sequence of a peptide obtained from the band of molecular weight of 8.7 kDa (symbol 99) could not be obtained. From the fact, it was also suggested that the peptide bond at the C-terminal side of the 178th aspartic acid (aspartylprolyl bonds) was cleaved.

The N-terminal amino acid sequence of a peptide obtained from the band of molecular weight of 7.0 kDa (symbol 100) could not be obtained. From the fact, it was also suggested that the peptide bond at the C-terminal side of the aspartic acid (aspartylprolyl bonds) was cleaved.

### (Results of cleavage of CGMMV)

CGMMV is a protein consisting of 160 amino acids with acetylated N-terminal amino acid residue, and its total amino acid sequence has been reported.

A band corresponding to CGMMV was detected. (symbol 101)

The N-terminal amino acid sequence of a peptide obtained from the band of molecular weight of 7.1 kDa (symbol 102) was Pro-Ser-Asn-Pro-Thr-. Based on this sequence and apparent molecular weight, it was proved that the peptide bond at the C-terminal side of the 98th aspartic acid (aspartylprolyl bond) was cleaved.

The N-terminal amino acid sequence of a peptide obtained from the band of molecular weight of 9.2 kDa (symbol 103) could not be obtained. From the fact, it was suggested that the peptide bond at the C-terminal side of the 98th aspartic acid (aspartylprolyl bonds) was cleaved.

Thus, it is shown that in a polypeptide chain, under the reaction conditions used in the present example, when the residue placed to the C-terminal side of an aspartic acid is proline, the peptide bond at the C-terminal side of the aspartic acid (aspartylprolyl bond) is to be cleaved.

The above-described results are summarized as follows.

In the present invention, in order to cleave a protein or peptide, an organic acid represented by the general formula; CF3-(CF2)n-COOH, wherein n is an integer of 0 or larger, was allowed to act on the protein or peptide. By this means it was made possible to cleave a protein into peptide fragments having specific amino acid terminal ends.

In the above-described examples, as the organic acid represented by the general formula; CF3-(CF2)n-COOH, wherein n is an integer of 0 or larger, heptafluorobutyric acid (n=2) was used. It is apparent that similar effect can be achieved by the use of other organic acids having similar chemical properties and also being represented by the general formula; CF3-(CF2)n-COOH,wherein n is an integral number of 0 or larger, for example pentafluoropropionic acid (n=1).

The important points of the present invention are to overcome disadvantages in the prior art and to make it possible to cleave a protein into peptide fragments having specific amino acid terminal ends by allowing an organic acid represented by the general formula; CF3-(CF2)n-COOH, wherein n is an integral number of 0 or larger, to cleave a protein or peptide. Thus the method of the present invention for cleaving a protein or peptide is of great value in industrial field.

[LIST OF SEQUENCES]
Sequence No.: 1
Length of sequence: 16
Form of sequence: amino acid
Topology: linear chain
Type of sequence: peptide
Sequence
Glu-Ala-Asp-Lys-Ala-Asp-Val-Asn-Val-Leu-Thr-Lys-Ala-Lys-
1                5                   10
Ser-Gln,
15

Sequence No.: 2
Length of sequence: 10
Form of sequence: amino acid
Topology: linear chain
Type of sequence: peptide
Sequence
Lys-Arg-Pro-Hyp-Gly-Phe-Ser-Pro-Phe-Arg
1                5                   10
Sequence No.: 3
Length of sequence: 17
Form of sequence: amino acid
Topology: linear chain
Type of sequence: peptide

Sequence
Lys-Ile-Leu-Gly-Asn-Gln-Gly-Ser-Phe-Leu-Thr-Lys-Gly-Pro-
1 5 10
Ser-Lys-Leu
15
Sequence No.: 4
Length of sequence: 11
Form of sequence: amino acid
Topology: linear chain
Type of sequence: peptide
Sequence
Glp-Lys-Arg-Pro-Ser-Gln-Arg-Ser-Lys-Tyr-Leu
1 5 10
Sequence No.: 5
Length of sequence: 13
Form of sequence: amino acid
Topology: linear chain
Type of sequence: peptide
Sequence
Arg-Arg-Leu-Ile-Glu-Asp-Ala-Glu-Tyr-Ala-Ala-Arg-Gly
1 5 10
Sequence No.: 6
Length of sequence: 12
Form of sequence: amino acid
Topology: linear chain

Type of sequence: peptide
Sequence
Val-Ser-Ser-Asn-Ile-Ser-Glu-Asp-Pro-Val-Pro-Val
1 5 10

## Claims

1. A method of obtaining pepetide fragments wherein a fluorinated organic acid preferably of general formula CF₃(CF₂)ₙ.COOH is reacted with a protein or a peptide, n being zero or an integer.

2. A method as claimed in claim 1 wherein the organic acid is in the vapour phase.

3. A method as claimed in claim 1 or claim 2 wherein the organic acid is reacted with the protein or peptide in the presence of water vapour.

4. A method according to any preceding claim wherein the organic acid is heptafluorobutyric acid or pentafluoropropionic acid.

5. A method as claimed in any preceding claim wherein the concentration of the acid is selected to achieve selective cleavage of one or more peptide linkages.

6. A method as claimed in claim 5 wherein the reaction is continued to achieve consecutive degradation of peptide linkages.

7. A method as claimed in any preceding claim wherein the pepetide is an an amino acid having a hydroxyl group in the beta position and a peptide linkage at the amino-terminal end of the amino acid is cleaved.

8. A method as claimed in any of claims 1 to 6 wherein the peptide is aspartic acid and a peptide linkage at its carboxy terminal end is cleaved.

9. A method as claimed in any of claims 1 to 6 wherein the peptide is threonine and a peptide linkage at its amino terminal end is cleaved.

10. A method as claimed in any of claims 1 to 6 wherein the peptide is serine and a peptide linkage at the amino terminal end is cleaved.


### Annex to the description

SEQUENCE LISTING

(1) GENERAL INFORMATION:

(i) APPLICANTS:
(A) NAME: SEIKO INSTRUMENTS INC.

(ii) TITLE OF INVENTION: Method for obtaining peptide
fragments

(iii) NUMBER OF SEQUENCES: 6

(v) COMPUTER READABLE FORM:
(A) MEDIUM TYPE: Floppy disk
(B) COMPUTER: PC
(C) OPERATING SYSTEM: OS2
(D) SOFTWARE: Text Editor

(vi) CURRENT APPLICATION DATA:

(A) APPLICATION NUMBER: EP97310175

(2) INFORMATION FOR SEQ ID NO:1:

(i) SEQUENCE CHARACTERISTICS:
(A) LENGTH: 16
(B) TYPE: amino acid
(D) TOPOLOGY: linear

(ii) MOLECULE TYPE: peptide

(xi) SEQUENCE DESCRIPTION:SEQ ID NO:1:

Glu Ala Asp Lys Ala Asp Val Asn Val Leu Thr Lys Ala Lys Ser Gln
1               5                   10                  15

(2) INFORMATION FOR SEQ ID NO:2:

(i) SEQUENCE CHARACTERISTICS:
(A) LENGTH: 10
(B) TYPE: amino acid
(D) TOPOLOGY: linear

(ii) MOLECULE TYPE: peptide

(ix) FEATURE:
(A) NAME/KEY : Mod_res
(B) LOCATION: 4
(D) OTHER INFORMATION :/note= Pro is Hydroxyproline

(xi) SEQUENCE DESCRIPTION:SEQ ID NO:2:

Lys Arg Pro Pro Gly Phe Ser Pro Phe Arg
1               5                   10

(2) INFORMATION FOR SEQ ID NO:3:

(i) SEQUENCE CHARACTERISTICS:
(A) LENGTH: 17

(B) TYPE: amino acid
(D) TOPOLOGY: linear

(ii) MOLECULE TYPE: peptide

(xi) SEQUENCE DESCRIPTION:SEQ ID NO:3:

Lys Ile Leu Gly Asn Gln Gly Ser Phe Leu Thr Lys Gly Pro Ser Lys
1               5                   10                  15

Leu

(2) INFORMATION FOR SEQ ID NO:4:

(i) SEQUENCE CHARACTERISTICS:
(A) LENGTH: 11
(B) TYPE: amino acid
(D) TOPOLOGY: linear

(ii) MOLECULE TYPE: peptide

(ix) FEATURE:
(A) NAME/KEY : Mod_res
(B) LOCATION: 1
(D) OTHER INFORMATION :/note= Glu is pyroglutamic acid

(xi) SEQUENCE DESCRIPTION:SEQ ID NO:4:

Glu Lys Arg Pro Ser Gln Arg Ser Lys Tyr Leu
1               5                   10

(2) INFORMATION FOR SEQ ID NO:5:

(i) SEQUENCE CHARACTERISTICS:
(A) LENGTH: 13
(B) TYPE: amino acid
(D) TOPOLOGY: linear

(ii) MOLECULE TYPE: peptide

(xi) SEQUENCE DESCRIPTION:SEQ ID NO:5:

Arg Arg Leu Ile Glu Asp Ala Glu Tyr Ala Ala Arg Gly
1               5                   10

(2) INFORMATION FOR SEQ ID NO:6:

(i) SEQUENCE CHARACTERISTICS:
(A) LENGTH: 12
(B) TYPE: amino acid
(D) TOPOLOGY: linear

(ii) MOLECULE TYPE: peptide

(xi) SEQUENCE DESCRIPTION:SEQ ID NO:6:

Val Ser Ser Asn Ile Ser Glu Asp Pro Val Pro Val
1               5                   10